(19) Europäisches Patentamt
European Patent Office
Office européen des brevets

(11) **EP 4 559 375 A1**

(12) **EUROPEAN PATENT APPLICATION**

(43) Date of publication:
**28.05.2025 Bulletin 2025/22**

(21) Application number: **24214062.2**

(22) Date of filing: **20.11.2024**

(51) International Patent Classification (IPC):
**A61B 1/00** (2006.01)     **A61B 1/005** (2006.01)
**A61M 25/01** (2006.01)     **A61B 34/30** (2016.01)

(52) Cooperative Patent Classification (CPC):
**A61B 1/00006; A61B 1/0016; A61B 1/0051;
A61B 1/009; A61M 25/01;** A61B 2034/301;
A61B 2034/302

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **21.11.2023 KR 20230161853**

(71) Applicant: **Medintech Inc.**
**Seoul 03100 (KR)**

(72) Inventors:
• **LEE, Chiwon**
  **12249 Namyangju-si, Gyeonggi-do (KR)**
• **KIM, Myungjoon**
  **13831 Gwacheon-si, Gyeonggi-do (KR)**
• **KIM, Geono**
  **02829 Seoul (KR)**

(74) Representative: **Isarpatent**
**Patent- und Rechtsanwälte**
**Barth Hassa Peckmann & Partner mbB**
**Friedrichstraße 31**
**80801 München (DE)**

(54) **METHOD, COMPUTER PROGRAM, AND APPARATUS FOR CONTROLLING ENDOSCOPIC SCOPE**

(57) According to one embodiment of the present disclosure, there is disclosed a method of controlling an endoscopic scope that is performed by a computing device including at least one processor. The method includes modeling the force required to control an endoscopic scope to provide power to the scope; identifying at least one parameter constituting a force model based on data on the shape or movement of the scope; and controlling the scope by providing the force, calculated based on the force model, to the scope.

FIG.1

EP 4 559 375 A1

**Description**

CROSS-REFERENCE TO RELATED APPLICATION

This application claims the benefit of Korean Patent Application No. 10-2023-0161853 filed on November 21, 2023, which is hereby incorporated by reference herein in its entirety.

BACKGROUND

1. Technical Field

The present disclosure relates to endoscope control technology, and more particularly, to a method, computer program, and apparatus for controlling an endoscopic scope.

2. Description of the Related Art

Endoscopes collectively refer to medical devices that enable a scope to be inserted into the human body and a user to observe an organ without surgery or autopsy. Endoscopes enable a scope to be inserted into the human body, light to be radiated, and the light reflected from the surface of the inner wall of the human body to be visualized. Endoscopes are classified according to their purpose and target body part, and may be basically classified into rigid endoscopes, in which an endoscopic tube is made of metal, and flexible endoscopes, which are represented by digestive endoscopes.
A flexible endoscope contains various devices therein and is thus vulnerable to impact, and also the inside of a digestive organ into which a flexible endoscope is inserted corresponds to a considerably fragile tissue and has an irregular shape. Furthermore, the shape of the inside of the digestive organ varies depending on the patient, so that the process of inserting the endoscope may not be easy even for experienced medical professionals.
In this case, as an endoscopic procedure is performed, an endoscopic scope is inserted into a digestive organ while being twisted and turned according to the shape of the digestive organ. In the early stage of the endoscopic procedure, an endoscopic operator may bend or move the scope to a desired angle without a large amount of force because the scope has a relatively simple shape. However, as the procedure progresses to the latter half of the procedure or when complex movement is involved during the procedure, controlling the scope while taking into consideration the characteristics of the scope that may change at any moment can cause significant inconvenience to the endoscopic operator.
In addition, the nature of the endoscopic procedure in which the scope is inserted into the body requires the delicate control of the scope, and thus, there is a demand for technology for controlling an endoscopic scope by reflecting therein the physical changes that occur in an endoscope device during an endoscopic procedure.

SUMMARY

The present disclosure is intended to overcome the problems of the above-described conventional art, and is directed to a method, computer program, and apparatus for generating a force model configured to control an endoscopic scope and then controlling an endoscopic scope based on the force model.
However, objects to be achieved by the present disclosure are not limited to the object described above, and another object may be present.
According to one embodiment of the present disclosure for achieving the above-described object, there is disclosed a method of controlling an endoscopic scope that is performed by a computing device including at least one processor. The method includes modeling the force required to control an endoscopic scope to provide power to the scope; identifying at least one parameter constituting a force model based on data on the shape or movement of the scope; and controlling the scope by providing the force, calculated based on the force model, to the scope.
Alternatively, the force required to control the scope may be related to the shape or movement of the scope.
Alternatively, the at least one parameter may include a first parameter related to the elasticity of the shape of the scope.
Alternatively, the force required to control the scope may include the frictional force of the scope.
Alternatively, the force required to control the scope may include the frictional force of a motor that provides power to the scope.
Alternatively, the at least one parameter may include a second parameter related to the friction of a wire within the scope.
Alternatively, the at least one parameter may include a third parameter related to the friction between components of the motor.

Alternatively, identifying the at least one parameter may include obtaining actual and estimated values of the force model and identifying at least one parameter that minimizes the residual difference between the actual and estimated values.

Alternatively, the at least one parameter constituting the force model may include a fourth parameter related to an assembly state of the scope.

Alternatively, the fourth parameter may be determined according to the angle of the scope and backlash.

Alternatively, the force model may be represented by a function of the angle of the scope, the speed of the scope, the acceleration of the scope, and the speed of a motor that provides the power.

According to one embodiment of the present disclosure for achieving the above-described object, there is disclosed a computer program stored in a computer-readable storage medium, the computer program performing operations for controlling an endoscopic scope when executed on one or more processors, wherein the operations include operations of: modeling the force required to control an endoscopic scope to provide power to the scope; identifying at least one parameter constituting a force model based on data on the shape or movement of the scope; and controlling the scope by providing the force, calculated based on the force model, to the scope.

According to one embodiment of the present disclosure for achieving the above-described object, there is disclosed a computing device for controlling an endoscopic scope, the computing device including: a processor including at least one core; and memory including a force model, in which at least one parameter has been identified, and program codes executable on the processor; wherein the processor controls an endoscopic scope by providing the force, calculated based on the force model, to the scope; and wherein the force model includes the force required to control the scope to provide power to the scope.

According to one embodiment of the present disclosure, there is generated the force required to assist the movement of an endoscopic scope by taking into consideration the physical characteristics of the scope that is twisted or moves in a complex shape during an actual endoscopic procedure, so that the accurate and precise control of the scope can be performed.

Accordingly, due to the nature of the endoscopic procedure that requires precise control, an endoscopic scope is controlled by taking into consideration nonlinearity, so that the error between the operation of an endoscopic operator and the movement of the scope is reduced, with the result that the convenience and satisfaction of the operator for the procedure are increased and higher stability can be provided from a patient's perspective.

## BRIEF DESCRIPTION OF THE DRAWINGS

The above and other objects, features, and advantages of the present invention will be more clearly understood from the following detailed description taken in conjunction with the accompanying drawings, in which:

FIG. 1 is a diagram showing the configuration of endoscope device according to one embodiment of the present disclosure;

FIG. 2 is an exemplary diagram showing a force model according to one embodiment of the present disclosure;

FIG. 3 is a flowchart showing a method of controlling an endoscopic scope according to one embodiment of the present disclosure; and

FIG. 4 is an exemplary diagram illustrating the estimation of a force model according to one embodiment of the present disclosure.

## DETAILED DESCRIPTION

Embodiments of the present disclosure will be described in detail below with reference to the accompanying drawings so that those having ordinary skill in the art of the present disclosure (hereinafter referred to as those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, a singular form should generally be construed to include "one or more."

The term "N-th (N is a natural number)" used herein can be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

Meanwhile, the term "module" or "unit" used herein may be understood as a term referring to an independent functional unit that processes computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a neural network "model" may refer to an overall system implemented as a neural network that is provided with problem-solving capabilities through training. In this case, the neural network may be provided with problem-solving capabilities by optimizing parameters connecting nodes or neurons through training. The neural network "model" may include a single neural network, or a neural network set in which multiple neural networks are combined together.

The foregoing descriptions of the terms are intended to help to understand the present disclosure. Accordingly, it should be noted that unless the above-described terms are explicitly described as limiting the content of the present disclosure, the terms in the content of the present disclosure are not used in the sense of limiting the technical spirit of the present disclosure.

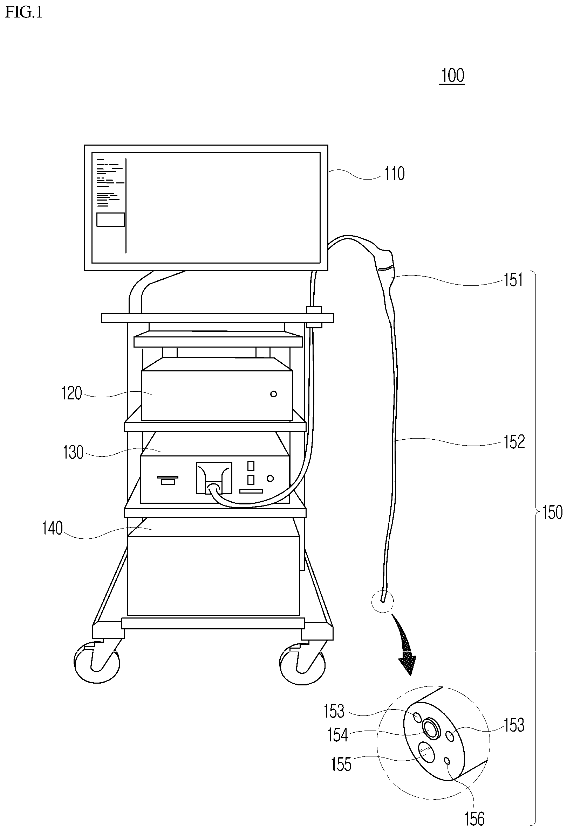

FIG. 1 is a diagram showing the configuration of endoscope device according to one embodiment of the present disclosure.

Referring to FIG. 1, an endoscope device 100 according to one embodiment of the present disclosure may be a flexible endoscope, or more specifically, a digestive endoscope. The endoscope device 100 may include a configuration capable of obtaining a medical image adapted to photograph the inside of a digestive organ and a configuration capable of, when necessary, allowing a tool to be inserted and a user to perform treatment or manipulation while viewing a medical image.

The endoscope device 100 may include an output unit 110, a control unit 120, a drive unit 130, a pump unit 140, and a scope 150, and may further include a light source unit (not shown).

The output unit 110 may include a display configured to display medical images. The output unit 110 may include a display module configured to output visualized information or implement touch screens, such as a liquid crystal display (LCD), a thin film transistor-liquid crystal display (TFT LCD), an organic light-emitting diode (OLED), a flexible display, a three-dimensional (3D) display, or the like.

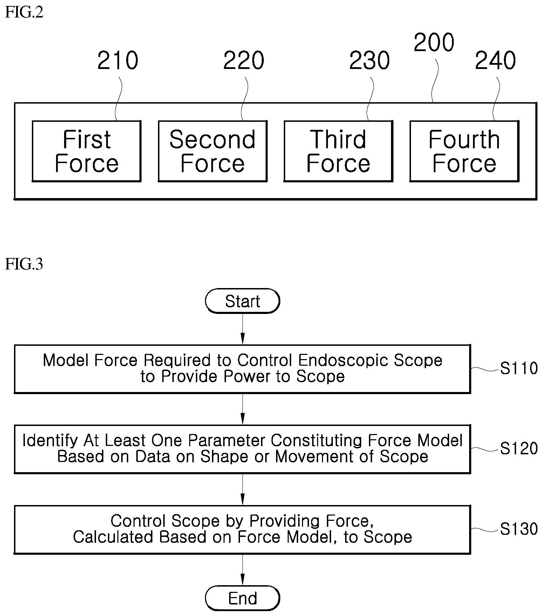

The output unit 110 may include various means for providing medical images or information about medical images. The output unit 110 may display medical images obtained by the scope 150 or medical images processed by the control unit 120. The output unit 110 may provide information through an auditory means in addition to a visual means, and may include, for example, a speaker configured to provide alarms regarding medical images audibly. Meanwhile, although the one output unit 110 is illustrated in FIG. 2, a plurality of output units 110 may be provided. In this case, an output unit 110 where a medical image obtained by the scope 150 is displayed and an output unit 110 where information processed by the control unit 120 is displayed may be separated from each other.

The control unit 120 may control the overall operation of the endoscope device 100. For example, the control unit 120 may perform the operation of taking a medical image through the scope 150, the operation of processing an obtained medical image, the control operation of performing medical operations such as the spraying of washing water, suction, or the like, a series of operations for controlling the movement of the scope 150, etc. The control unit 120 may include all types of devices capable of processing data. According to an exemplary embodiment, the control unit 120 may be a data processing device embedded in hardware that has circuits physically structured to perform the functions represented by the codes or instructions included in a program. As an example of the data processing device embedded in hardware, a processing device such as a microprocessor, a central processing unit (CPU), a processor core, a multiprocessor, an application-specific integrated circuit (ASIC), or a field programmable gate array (FPGA) may be included, but the technical spirit of the present disclosure is not limited thereto.

The endoscope device 100 of the present invention may include a computing device including a processor and memory. For example, the computing device may constitute the control unit 120 of the endoscope device 100. That is, the computing device may be configured to perform the operation of the control unit 120.

The processor according to an embodiment of the present disclosure may be understood as a constituent unit including hardware and/or software for performing computing operation. For example, the processor may read a computer program and perform data processing for machine learning. The processor may process computational processes such as the processing of input data for machine learning, the extraction of features for machine learning, and the calculation of errors based on backpropagation. The processor for performing such data processing may include a central processing unit (CPU), a general purpose graphics processing unit (GPGPU), a tensor processing unit (TPU), an application specific integrated circuit (ASIC), or a field programmable gate array (FPGA). Since the types of processor described above are only examples, the type of processor may be configured in various manners within a range understandable to those skilled in the art based on the content of the present disclosure.

The memory according to an embodiment of the present disclosure may be understood as a constituent unit including hardware and/or software for storing and managing data that is processed in the computing device. That is, the memory may store any type of data generated or determined by the processor and any type of data received by the network unit. For example, the memory may include at least one type of storage medium of a flash memory type, hard disk type, multimedia card micro type, and card type memory, random access memory (RAM), static random access memory (SRAM), read-only memory (ROM), electrically erasable programmable read-only memory (EEPROM), programmable read-only memory (PROM), magnetic memory, a magnetic disk, and an optical disk. Furthermore, the memory may include a database system that controls and manages data in a predetermined system. Since the types of memory described above are only examples, the type of memory may be configured in various manners within a range understandable to those skilled in the art based on the content of the present disclosure. The memory may be a non-transitory medium configured to store data and instructions in a tangible form, and is not a mere signal or transitory form.

The memory may structure, organize and then manage data required for the processor to perform operations, combinations of the data, and program codes executable on the processor. Furthermore, the memory may also store program codes adapted to operate the processor to generate training data.

The computing device may further include a network unit configured to transmit and receive data to and from an external computing device, another endoscope device, a hospital server, or the like.

The data to be processed by the processor may be stored in the memory or received over a network, and the data generated by the processor may be stored in the memory or transmitted to the outside over a network.

The control unit 120 may control the movement of the scope 150 through the drive unit 130 connected to the scope 150. That is, the control unit 120 may generate control signals to be provided to the drive unit 130 in order to control the movement of the scope 150.

For example, a series of operations in which the endoscope device 100 of the present disclosure controls the scope 150 may be performed as follows. A user may input the degree or direction of curvature of the scope 150 through a manipulation portion 151. The input information is transmitted to the control unit 120, and the control unit 120 may process the input information and generate a signal to be provided to the drive unit 130. For example, the control unit 120 may calculate the position, angle, angular velocity, and/or like of a motor corresponding to the degree or direction of curvature set by the user and may provide them to the drive unit 130. The drive unit 130 may generate power based on the signal of the control unit 120 and transmit it to the scope 150. Accordingly, the scope 150 may be moved or bent in accordance with the value input by the user.

The endoscope device 100 according to the present disclosure may model the force for controlling the scope 150, may calculate the force to be provided to the scope 150 by using a force model generated as a result of the modeling, and may provide the calculated force to the scope 150. In this case, the endoscope device 100 may generate the force model by taking into consideration nonlinear characteristics that occur in a system including the scope 150. To control the scope 150 based on the degree or direction of curvature input through the manipulation portion 151, the endoscope device 100 may compensate the force to be provided to the scope 150 using data on the shape or movement of the scope 150.

The endoscope device 100 may determine a plurality of forces that constitute the force model, and may identify parameters for defining the respective forces. The plurality of forces may be obtained by modeling the physical shape and mechanical structure of the scope 150, the characteristics of the drive unit 130 configured to provide power to the scope 150, the relationship between elements that constitute the drive unit 130, errors that occur during the process of assembling the scope 150, and/or the like. For example, the endoscope device 100 may use an optimization technique to identify the parameters of the force model, but the parameter identification method is not limited thereto.

The endoscope device 100 may calculate the force to be provided to the scope 150 by using the force model, in which parameters have been identified, and at least one of the target angle of the scope 150, the target speed of the scope 150, the target acceleration of the scope 150, and the target speed of the motor for the control of the endoscopic scope 150. In this case, the calculated force may be generated from the motor connected to the scope 150.

According to the present disclosure, the force intended to assist the movement of the scope 150 is generated by taking into consideration the physical characteristics of the scope 150 that is twisted or moved in a complex shape during an actual endoscopic procedure, so that the accurate and precise control of the scope 150 can be performed.

In addition, the scope 150 is controlled by taking into consideration various types of nonlinearity that may occur in the endoscopic system, so that the error between the operation of an endoscopic operator and the movement of the scope 150 is reduced, and accordingly, with the result that the convenience and satisfaction of the endoscopic operator can be increased.

The drive unit 130 may provide power required for the scope 150 to be inserted into the body, bent, and moved inside the body. For example, the drive unit 130 may include the motor connected to the wire inside the scope 150 and a tension adjustment unit configured to adjust the tension of the wire.

The drive unit 130 may control the scope 150 in various directions by controlling the power of the motor. For example, the motor may be configured to include a plurality of motors corresponding to the directions in which the insertion portion 152 at the end of the scope 150 is to be bent. Alternatively, the motor may be configured to include a plurality of motors corresponding to wires inside the scope 150. More specifically, the drive unit 130 may include a first motor configured to determine the x-axis movement of the scope 150 and a second motor configured to determine the y-axis movement of the scope 150. The x-axis position, y-axis position, z-axis position, roll, pitch, and yaw values of the end of the scope 150 may be determined according to the control of the drive unit 130, but the configuration of the drive unit 130 is not limited thereto.

The tension adjustment unit may receive power from the motor and pull the wire inside the scope 150 to generate tension. Through this, the scope 150 may be bent. The tension adjustment unit 330 may adjust the tension applied to the plurality of wires 1000 inside the scope 150 so that the scope 150 can be bent according to the determined amount and direction of bending.

The pump unit 140 may include at least one of an air pump configured to inject air into the body through the scope 150, a suction pump configured to provide negative pressure or vacuum and suck air from the body through the scope 150, and a water pump configured to inject cleaning water into the body through the scope 150. Each of the pumps may include a valve configured to control the flow of fluid. The pump unit 140 may be opened and closed by the control unit 120. At least one of the suction pump, the water pump, and the air pump may be opened and closed based on a control signal of a computing device or the control of the control unit 120.

The scope 150 may include the insertion portion 152 configured to be inserted into a digestive organ and the operating portion 151 configured to control the movement of the insertion portion 152 and receive input from a user to perform various operations.

The insertion portion 152 is configured to be flexibly bent and is connected to the drive unit 130 at one end thereof, so that the degree or direction of curvature can be determined by the drive unit 130. Since medical imaging and treatment are performed at the end of the insertion portion 152, the scope 150 may include various cables and tubes that extend to the end of the insertion portion 152. A light source lens 153, an objective lens 154, a working channel 155, and an air and water channel 156 may be provided inside the scope 150. A tool for treating and managing a lesion may be inserted through the working channel 155 during an endoscopic procedure. Air may be injected and washing water may be fed through the air and water channel 156. Meanwhile, in FIG. 2, the air and water channel 156 is illustrated as a passage through which washing water is fed, but it is not limited thereto. For example, a separate water jet channel (not shown) may be provided inside the scope 150, and cleaning water may also be fed through a water jet channel.

Meanwhile, references herein to the scope 150 being bent by the control unit 120 or the drive unit 130 may refer to at least a part of the scope 150, e.g., the insertion portion 152, being bent.

The manipulation portion 151 may include a plurality of input buttons configure to provide various functions (image capture, the spray of cleaning water, etc.) to allow an endoscopic operator to control the steering of the insertion portion 152 and perform a procedure through the working channel 155 and the air and water channel 156. For example, the manipulation portion 151 may include a plurality of buttons or a joystick-type input device configured to indicate the direction of the scope 150.

The light source unit may include a light source that radiates light into the body through the endoscopic scope 150. The light source unit may include a lighting device configured to generate white light, or may include a plurality of lighting devices configured to generate rays of light having different wavelength bands. The type of light source, the intensity of light, white balance, and/or the like may be set through the light source unit. Meanwhile, the above-described setting items may also be set through the control unit 120. The light generated by the light source unit may be transmitted to the scope 150 through a path such as an optical fiber.

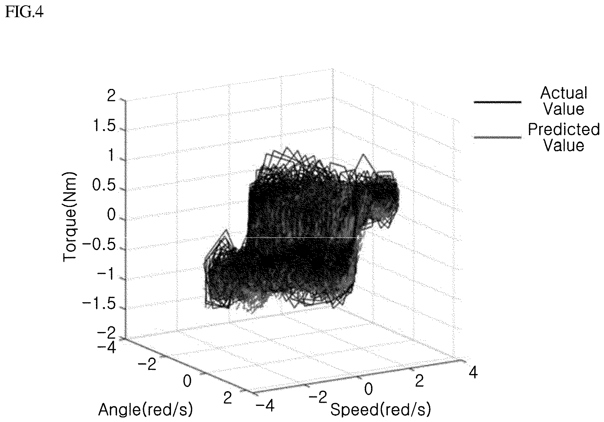

FIG. 2 is an exemplary diagram showing a force model according to one embodiment of the present disclosure, FIG. 3 is a flowchart showing a method of controlling an endoscopic scope according to one embodiment of the present disclosure, and FIG. 4 is an exemplary diagram illustrating the estimation of a force model according to one embodiment of the present disclosure.

Referring to FIGS. 2 and 3 together, the computing device may model the force intended to control the scope 150 to provide power to the endoscopic scope 150 in step S110. The computing device may receive the target curvature, curvature direction, curvature angle, and/or like of the scope 150 from a user through the manipulation portion 151. The computing device may model the force intended to control the scope 150 based on the received values. In this case, the force intended to control the scope 150 may be related to the shape or movement of the scope 150. Furthermore, the force intended to control the scope 150 may be generated from the drive unit 130 connected to the wire within the scope 150. In other words, the force to be modeled may be the torque generated from the motor. For example, the force model 200 may be represented by a function of the angle of the scope 150, the speed of the scope 150, the acceleration of the scope 150, and the speed of the motor providing power.

Referring to FIG. 2, the force model 200 obtained by the modeling of the computing device may include first to fourth forces 210, 220, 230, and 240. For example, the first force 210 may include a force related to the shape of the scope 150, the second force 220 may include a force related to the friction of the wire within the scope 150, and the third force 230 may include a force related to the friction between components of the drive unit 130. Furthermore, the fourth force 240 may include a dynamics term intended to compensate for nonlinearity, and may include, e.g., a sliding mode control dynamics term.

More specifically, the first to fourth forces 210, 220, 230, and 240 may be modeled in the form of Formulas 1 to 4 below, respectively. In this case, $\theta_s^{act}$ may denote the actual angle of the scope 150, $\dot{\theta}_s^{act}$ may denote the actual velocity of the scope 150, and $\ddot{\theta}_s^{act}$ may denote the actual acceleration of the scope 150.

$$K\theta_s^{act\,2} \qquad\qquad (1)$$

where K may denote a first parameter related to the elasticity of the shape of the scope 150.

That is, the computing device according to the present disclosure calculates the force by compensating for the nonlinear characteristics occurring in an actual endoscope by modeling the elastic force generated due to the twisting of the scope 150 that occurs while an endoscopic procedure is performed.

$$f_{s,c}\,sign(\theta_s^{act}) \qquad\qquad (2)$$

The second force 220 shown in Formula 2 is related to the static friction and dynamic friction generated within the scope 150. The second force 220 has a discontinuous point occurring at 0, and may be represented by a coulomb-viscous friction model, including a linear gain, at the remaining points excluding 0. $f_{s,c}$ is a second parameter. The second force 220 may be modeled only when the second parameter is identified.

That is, the computing device according to the present disclosure may reflect the nonlinear characteristics occurring in the endoscope therein by modeling the frictional force of the wire inside the scope 150 that is generated due to the complex shape of the scope 150 that occurs while an endoscopic procedure is performed.

$$f_{stribeck} \qquad\qquad (3)$$

The third force 230 shown in Formula 3 may be represented by a stribeck model that reflects therein changes in frictional force according to the speed. More specifically, the third force 230 may be represented by the following formula:

$$f_{stribeck} = f_{m,c} + \left(f_{m,s} - f_{m,c}\right)sign(w)e^{-\frac{w}{w_s}^{\sigma}}, w = \dot{\theta}_m^{act}$$

The third force 230 includes a third parameter, and the third parameter may include $f_{m,c}$, $f_{m,s}$, and $f_{m,c}$. That is, the computing device according to the present disclosure may provide more accurate power to the scope 150 by modeling the frictional force generated from the motor to operate the scope 150 that occurs while an endoscopic procedure is performed.

$$C\dot{\theta}_s^{act} + M\ddot{\theta}_s^{act} \qquad (4)$$

The fourth force 240 shown in Formula 4 may include a fourth parameter including C and M. In this case, C may denote a parameter reflecting therein the Coriolis effect occurring in the scope 150, and $M$ may denote a parameter reflecting inertia therein.

Meanwhile, although not shown in FIG. 2, the force model 200 may further include a parameter intended to compensate the force generated from the drive unit 130. For example, the force model 200 may further include a fifth parameter related to the assembly state of the scope 150. When the scope 150 is assembled, there may be a difference in the assembly state depending on the skill level of the worker. Accordingly, the computing device needs to generate forces intended to control the scope 150 by adjusting control parameters, parameters, etc. with the assembly characteristics of the scope 150 reflected therein. As a result, even when the assembly state changes, an endoscopic operator may feel a consistent sense of manipulation. Therefore, the force model 200 may further include a correction coefficient $\alpha$ as a parameter for reflecting the assembly state therein. The correction coefficient may be multiplied by the force generated by the force model 200. The correction coefficient may be determined according to the angle of the scope 150 and backlash. In the present disclosure, the term "backlash" refers to a phenomenon in which the force generated from the drive unit 130 is transmitted through a wire inside the scope 150 but the movement of the end of the scope 150 does not occur.

More specifically, although the size of the correction coefficient may be determined according to the maximum backlash angle, the size of the correction coefficient is not limited thereto. However, it is sufficient if it is a numerical value that reflects the assembled state therein.

The computing device may identify at least one parameter constituting the force model 200 based on data on the shape or movement of the scope 150 in step S 120. The computing device may obtain the actual value and estimated value of the force model 200, and may identify at least one parameter that minimizes the residual difference between the actual value and the estimated value.

Referring to FIG. 4, the black curve represents actual acquired values and the gray curve represents estimated values. For example, the computing device may derive a parameter that allows the difference between the actual acquired value and the estimated value to fall within a preset range through an optimal control technique.

The computing device may control the scope 150 by providing the force, calculated based on the force model 200, to the scope 150 in step S 130. The computing device may preemptively identify the states of the scope 150 and the motor based on the force model 200 including the identified parameter. Accordingly, the torque required by the motor may be generated. In this case, the torque may play the role of a feedforward torque used to offset the nonlinearity of the endoscope device 100. This may minimize errors occurring in the feedback control process performed in the endoscope device 100.

According to the present disclosure, when the scope 150 moves within the body, a minute difference in the movement may cause lesion detection to be missed or have a fatal effect on the endoscopic procedure. Therefore, due to the nature of the endoscopic procedure that requires precise control, the scope 150 is controlled by taking into consideration nonlinearity, so that the accuracy and convenience of the endoscopic procedure are increased and higher stability is also provided from a patient's perspective.

The description of the present disclosure described above is intended for illustrative purposes, and those of ordinary skill in the art to which the present disclosure pertains can appreciate that the present disclosure may be easily modified into other specific forms without changing the technical spirit or essential features of the present disclosure. Therefore, it should be understood that the embodiments described above are illustrative and not restrictive in all respects. For example, each component described as being in a single form may be implemented in a distributed form. In the same manner, components described as being in a distributed form may be implemented in a combined form.

The scope of the present disclosure is defined by the following claims rather than the above detailed description, and all changes or modifications derived from the meanings and scope of the claims and their equivalent concepts should be interpreted as being encompassed within the scope of the present disclosure.

**Claims**

1. A method of controlling an endoscopic scope, the method being performed by a computing device including at least one processor, the method comprising:

   modeling a force required to control an endoscopic scope to provide power to the scope;
   identifying at least one parameter constituting a force model based on data on a shape or movement of the scope; and
   controlling the scope by providing a force, calculated based on the force model, to the scope.

2. The method of claim 1, wherein the force required to control the scope is related to the shape or movement of the scope.

3. The method of claim 2, wherein the at least one parameter includes a first parameter related to elasticity of the shape of the scope.

4. The method of claim 3, wherein the force required to control the scope includes a frictional force of the scope.

5. The method of claim 4, wherein the force required to control the scope includes a frictional force of a motor that provides power to the scope.

6. The method of claim 4, wherein the at least one parameter includes a second parameter related to friction of a wire within the scope.

7. The method of claim 5, wherein the at least one parameter includes a third parameter related to friction between components of the motor.

8. The method of claim 1, wherein identifying the at least one parameter comprises obtaining actual and estimated values of the force model and identifying at least one parameter that minimizes a residual difference between the actual and estimated values.

9. The method of claim 1, wherein the at least one parameter constituting the force model further includes a fourth parameter related to an assembly state of the scope.

10. The method of claim 9, wherein the fourth parameter is determined according to an angle of the scope and backlash.

11. The method of claim 1, wherein the force model is represented by a function of an angle of the scope, a speed of the scope, an acceleration of the scope, and a speed of a motor that provides the power.

12. A computer program stored in a computer-readable storage medium, the computer program performing operations for controlling an endoscopic scope when executed on one or more processors,
    wherein the operations comprise operations of:

    modeling a force required to control an endoscopic scope to provide power to the scope;
    identifying at least one parameter constituting a force model based on data on a shape or movement of the scope; and
    controlling the scope by providing a force, calculated based on the force model, to the scope.

13. A computing device for controlling an endoscopic scope, the computing device comprising:

    a processor including at least one core; and
    memory including a force model, in which at least one parameter has been identified, and program codes executable on the processor;
    wherein the processor controls an endoscopic scope by providing a force, calculated based on the force model, to the scope; and
    wherein the force model includes a force required to control the scope to provide power to the scope.

FIG.1

EP 4 559 375 A1

FIG.2

FIG.3

FIG.4

Europäisches
Patentamt
European
Patent Office
Office européen
des brevets

# EUROPEAN SEARCH REPORT

**Application Number**

EP 24 21 4062

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/030023 A1 (YOSHIE MICHIFUMI [JP]) 4 February 2010 (2010-02-04) * paragraphs [0003], [0038], [0060], [0064], [0065], [0067], [0068], [0071], [0079], [0080], [0141]; figures 1, 2 * | 1-13 | INV. A61B1/00 A61B1/005 A61M25/01 ADD. A61B34/30 |
| X | US 2019/335981 A1 (HANE JUN [JP]) 7 November 2019 (2019-11-07) * paragraphs [0006], [0061], [0141], [0142], [0285], [0330], [0310]; figure 1 * | 1-13 | |
| X | CN 114 452 507 A (SHANGHAI MINIMALLY INVASIVE MICRO AVIATION ROBOT LTD COMPANY) 10 May 2022 (2022-05-10) * paragraphs [0111], [0121], [0126], [0132], [0140] - [0143], [0145], [0257], [0285] * | 1-13 | |

**TECHNICAL FIELDS SEARCHED (IPC)**

A61B
A61M

The present search report has been drawn up for all claims

| Place of search | Date of completion of the search | Examiner |
|---|---|---|
| Munich | 9 April 2025 | Höß, Theresa |

EPO FORM 1503 03.82 (P04C01)

## ANNEX TO THE EUROPEAN SEARCH REPORT
## ON EUROPEAN PATENT APPLICATION NO.

EP 24 21 4062

This annex lists the patent family members relating to the patent documents cited in the above-mentioned European search report.
The members are as contained in the European Patent Office EDP file on
The European Patent Office is in no way liable for these particulars which are merely given for the purpose of information.

09-04-2025

| Patent document cited in search report | Publication date | Patent family member(s) | | Publication date |
|---|---|---|---|---|
| US 2010030023 A1 | 04-02-2010 | CN | 101642364 A | 10-02-2010 |
| | | EP | 2151184 A1 | 10-02-2010 |
| | | JP | 2010035768 A | 18-02-2010 |
| | | US | 2010030023 A1 | 04-02-2010 |
| US 2019335981 A1 | 07-11-2019 | CN | 110191668 A | 30-08-2019 |
| | | US | 2019335981 A1 | 07-11-2019 |
| | | WO | 2018134898 A1 | 26-07-2018 |
| | | WO | 2018135018 A1 | 26-07-2018 |
| CN 114452507 A | 10-05-2022 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230161853